# EUROPEAN PATENT SPECIFICATION

(11) **EP 1 432 441 B1**
(45) Date of publication and mention of the grant of the patent: **29.07.2009**
(21) Application number: 02775057.9
(22) Date of filing: 25.09.2002
(51) Int. Cl.: A61K 39/39, A61K 38/17, A61P 37/02

(54) **USE OF HMGB1 FOR THE ACTIVATION OF DENDRITIC CELLS**
VERWENDUNG VON HMGB1 ZUR AKTIVIERUNG DENDRITISCHER ZELLEN
UTILISATION DE HMGB1 POUR L'ACTIVATION DE CELLULES DENDRITIQUES

(30) Priority: 25.09.2001 IT MI20011986
(43) Date of publication of application: 30.06.2004
(73) Proprietor: FONDAZIONE CENTRO SAN RAFFAELE DEL MONTE TABOR, 20132 Milano (IT)
(72) Inventor: BIANCHI, Marco, I-20132 Milan (IT); MANFREDI, Angelo, I-20132 Milan (IT)
(74) Representative: Mallalieu, Catherine Louise
(86) International application number: PCT/IB2002/004080
(87) International publication number: WO 2003/026691

(56) References cited:
- WO-A-00/47104
- WO-A-99/59609

## Description

### Field of the Invention

This present invention relates to a use of the protein HMGB1, or a polynucleotide encoding therefor, and particularly, but not exclusively, to methods of treating diseases associated with an antigen specific immune response, and to compositions comprising the HMGB1 protein or polynucleotide encoding therefor.

### Background to the Invention

The immune system protects the body from potentially harmful substances by recognising and responding to so-called antigens. Antigens are large molecules (usually proteins), either integral or surface constituents of cells, or viruses, fungi, or bacteria. Some non-living substances such as toxins, chemicals, drugs, and foreign particles can be antigens. Substances that contain these antigens are recognised and destroyed by the immune system. The immune system also learns to see antigens associated to MHC (HLA in humans) molecules as "normal" or "self" and does not usually react against them.

Acquired (adaptive) immunity develops when the body is exposed to various antigens and builds a defence that is specific to that antigen. Lymphocytes contain subgroups, B and T lymphocytes, that are key players in acquired immune responses. B lymphocytes (also called B cells) produce antibodies. Antibodies attach to a specific antigen and make it easier for the phagocytes to destroy the antigen. T lymphocytes (T cells) recognize MHC-associated antigens on the surface of cells, and provide control of the immune response. B cells and T cells develop that are specific for one antigen type. Thus when the immune system is exposed to a different antigen, different B cells and T cells are formed.

As lymphocytes develop, they normally learn to recognise the body's own tissues (self) as distinctive from tissues and particles not normally found in your body (non-self). Once B cells and T cells are formed, a few of those cells will multiply and provide "memory" for the immune system. This allows the immune system to respond faster and more efficiently the next time you are exposed to the same antigen, and in many cases will prevent you from getting sick. For example, adaptive immunity accounts for an individual who has had chickenpox for being so-called 'immune' to getting chickenpox again.

Vaccination (immunisation) is a way to trigger the immune response. Small doses of an antigen (such as dead or weakened live viruses) are given to activate immune system "memory" (activated B lymphocytes and sensitised T lymphocytes). Memory allows your body to react quickly and efficiently to future exposures. As noted above, this means that if you are exposed to a microorganism, it will be destroyed before it can cause illness.

Immune system disorders occur when the immune response is inappropriate, excessive, or lacking. Allergies involve an immune response to a substance that, in the majority of people, the body perceives as harmless. Transplant rejection involves the destruction of transplanted tissues or organs and is a major complication of organ transplantation. Blood transfusion reaction is a complication of blood administration. Autoimmune disorders (such as systemic lupus erythematosus and rheumatoid arthritis) occur when the immune system acts to destroy normal body tissues. Immunodeficiency disorders (such as inherited immunodeficiency and AIDS) occur when there is a failure in all or part of the immune system.

Thus, usually the activation of the immune response is desired. In some cases, suppression of the immune system is necessary (for example, in the treatment of autoimmune disorders or allergies). This is usually accomplished by administering corticosteroids or other immunosuppressive medications, but these have a general immune suppressive effect, i.e. are not antigen specific.

An efficient immune response therefore protects against many diseases and disorders. Inefficient immune response allows diseases to develop. Inadequate, inappropriate, or excessive immune response causes immune system disorders.

Complications related to altered immune response include:
- disease development
- allergy/hypersensitivity
- anaphylaxis
- autoimmune disorders
- blood transfusion reaction
- immunodeficiency disorders
- serum sickness
- transplant rejection
- graft versus host disease

Various ways of altering an immune response have been proposed but there is an ongoing need to provide ways of treating diseases such as those mentioned above.

The non-histone nuclear protein HMGB1 belongs to the B family of HMG proteins, also known as the high mobility group. It has recently been reported that the non-histone nuclear protein HMGB1 is released by necrotic cells (10). In living cells HMGB1 does not bind to chromatin in a stable fashion; on the other hand it is sequestrated by the nuclear chromatin deacetylated during apoptosis.

EP 1 079 849 discloses the use of HMG proteins for use as the cytotoxic agent in a pharmaceutical composition. In more detail it describes administering HMG-I as a cytotoxic agent to rats having tumors. HMG-I is now designated as HMGA1, i.e. from the HMG A family, which does not have any molecular similarity which the HMG B family. No evidence is provided in EP 1079 849 in relation to the activity of the B family.

In contrast to the teaching in EP 1079 849 we have now found that HMGB1 has no direct cytotoxic activity, but rather cooperates in activating immune responses. Therefore it may be used to elicit antigen-specific anti-tumor immune responses, and to complement the effect of an anti-neoplastic agent.

Extracellular HMGB1 determines the production of TNF-α and of other cytokines and is involved in the pathogenesis of septic shock (12,13, WO00/47104). Moreover, the concentration of HMGB1 increases during haemorrhagic shock in the absence of bacterial components (14). In more detail, WO00/47104 describes a pharmaceutical composition for treating conditions characterised by activation of the inflammatory cytokine cascade comprising an antagonist or inhibitor of HMG1 (now designated HMGB1). WO00/47104 gives a long list of conditions which it describes as being mediated by the inflammatory cytokine cascade. In contrast to the approach of WO00/47104, we have now found that HMGB1 can be used to regulate an antigen mediated immune response. For example, in the approach taught in WO00/47104 conditions such as some infectious diseases and some malignancies may be treated by using an antagonist of HMGB1. However, following the antigen specific immune response approach of the present invention we have found that administration of HMGB1 may be used.

The present invention thus provides a further method of treating a range of disorders associated with the acquired immune response.

### Summary of the Invention

In general terms the present invention relates to the modulation of the activation (maturation) of antigen presenting cells. In one embodiment the present invention relates to a method for inducing the activation (maturation) of antigen presenting cells, the compositions used for this purpose and their use in the activation of the immune response. In particular, it has now been found, surprisingly, that HMGB1 possesses a pronounced effect on induction of the maturation of dendritic cells.

### Statements of the Invention

According to one aspect of the present invention there is provided use of the protein HMGB1 or a biologically active fragment thereof, or a polynucleotide encoding therefor, for the preparation of a medicament for inducing the activation of an antigen presenting cell (APC), and wherein the medicament is for stimulating an immune response.

Preferably said APC is a dendritic cell.

In one embodiment the activation is carried out *in vitro,* but it may equally well take place *in vivo.*

Preferably said medicament is in the form of a vaccine.

Preferably the vaccine is for use in relation to a tumor, or bacterial or viral infection, more preferably it is an anti-cancer vaccine.

Preferably the HMGB1 acts as an adjuvant in the medicament.

Thus, according to another aspect of the present invention there is provided use of the protein HMGB1 or a biologically active fragment thereof, or a polynucleotide encoding therefor, for the preparation of a medicament for inducing the activation of an APC for use as an adjuvant.

According to another aspect of the present invention there is provided use of the protein HMGB1 or a biologically active fragment thereof, or a polynucleotide encoding therefor, for the preparation of a medicament for inducing the activation of an APC for the treatment or prevention of a bacterial or viral infection.

According to another aspect of the present invention there is provided use of an antibody or an antisense sequence to the protein HMGB1 or a biologically active fragment thereof, or a polynucleotide encoding therefor, for the preparation of a medicament for reducing activation of an APC.

In one embodiment said reducing activation is carried out *in vitro,* but it may equally well take place *in vivo.*

In other words the present invention provides use of an antibody or antisense sequence to the protein HMGB1 or a biologically active fragment thereof, or a polynucleotide encoding therefor, for the preparation of a medicament for downregulating an antigen specific immune response.

According to another aspect of the present invention there is provided use of an antibody or antisense sequence to the protein HMGB1 or a biologically active fragment thereof, or a polynucleotide encoding therefor, for the preparation of a medicament for the treatment of an inflammatory or autoimmune disease, allergy or transplant rejection.

Preferably the medicament is in the form of a vaccine.

According to another aspect of the present invention there is provided an *in vitro* method for producing an activated APC comprising exposing the APC to the protein HMGB1 or a variant or fragment thereof or a polynucleotide encoding therefor.

Preferably the APC is also exposed to an antigen.

Preferably the APC and/or antigen are also exposed to a T cell.

Preferably the antigen is a tumor, bacterial or viral antigen.

In another aspect the present invention also provides an *in vitro* method of reducing or preventing activation of an APC comprising exposing the APC to an antibody or antisense sequence to the protein HMGB1 or a biologically active fragment thereof, or a polynucleotide encoding therefor.

Preferably the APC is also exposed to an antigen.

Preferably the APC and/or antigen are also exposed to a T cell.

In one embodiment the antigen is an allergen or is associated with an inflammatory condition, autoimmune disease or transplant rejection.

In one embodiment the APC is transfected with a vector for the expression of an antigen or of an MHC molecule.

Preferably wherein the APC is a dendritic cell.

Preferably the HMGB 1 is in the form of a vaccine.

According to a further aspect of the present invention there is provided a pharmaceutical composition comprising the protein HMGB1 or a biologically active fragment thereof, or a polynucleotide encoding therefor, and an antigen.

Preferably the composition is in the form of a vaccine.

Preferably the pharmaceutical composition further comprises an APC.

### Detailed description of the Invention

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology:DNA Structure Part A: Synthesis and Physical Analysis of DNA Methods in Enzymology, Academic Press.

The present invention relates to the use of HMGB1, or of its biologically active fragments, or a polynucleotide encoding therefor, for the preparation of pharmaceutical compositions for stimulating the immune response to a particular antigen. Said compositions may be used in vaccination, in particular in vaccination against tumours or against infective agents. It will be possible for the HMGB1 protein or its fragments, or polynucleotides encoding therefor, suitably formulated, to be administered together with the antigen or separately from it, with the function of adjuvants.

The present invention also includes the use of antibodies directed against HMGB1, or against a fragment thereof, for the treatment of autoimmune or inflammatory pathologies. Blocking of the activity of HMGB1, in fact, impedes the maturation of dendritic cells in response to pro-inflammatory stimuli, and can be used in the treatment of chronic autoimmune and inflammatory diseases involving auto-reactive T cells or high-affinity antibodies (e.g. systemic or organ-specific autoimmune diseases, vasculitis).

Thus, the present invention generally relates to a method of modulating the immune response, particularly the adaptive or antigen specific immune response, and more particularly the present invention relates to the modulating of the activation of APCs. As used herein the terms "modulates"/"modulating" preferably mean any one or more of: adversely affecting, decreasing, removing, inhibiting, antagonising, blocking or down regulating activity.

### HMGB1

As indicated above, HMGB1 is a member of the B family of HMG proteins, also known as High Mobility Group proteins. HMGB1 is almost identical (about 99% amino acid identity) in mammals. Preferably the present invention employs human HMGB1. Rat HMGB1 is reported in Bianchi et al., 1989, Specific recognition of cruciform DNA by nuclear protein HMG1, Science 243: 1056-1059 (access No. of the sequence in the databank Y00463). Human HMGB1 and mouse HMGB1 are reported in several access numbers (for example NM_002128 for human and NM_010439 for mouse).

As reported in WO00/47104, HMG1 is a 30 kDa chromosomal nucleoprotein belonging to the burgeoning high mobility group (HMG) of non-histone chromatin-associated proteins. As a group, the HMG proteins recognize unique DNA structures and have been implicated in diverse cellular functions, including determination of nucleosome structure and stability, as well as in transcription and/or replication. The HMG proteins were first characterized by Johns and Goodwin as chromatin components with a high electrophoretic mobility in polyacrylamide gels (see in The HMG Chromosomal Proteins, E.W. Johns, Academic Press, London, 1982). Higher eukaryotes exhibit three families of HMG proteins; the HMG-1/-2 family, the HMG-14/-17 family and the HMG-1/-Y family. The families are distinguishable by size and DNA-binding properties. HMG proteins are highly conserved across species, ubiquitously distributed and highly abundant, and are extractable from chromatin in 0.35 M NaCl and are soluble in 5% perchloric or trichloroacetic acid. Generally, HMG proteins are thought to bend DNA and facilitate binding of various transcription factors to their cognate sequences, including for instance, progesterone receptor, estrogen receptor, HOX proteins, and Oct1, Oct2 and Oct6. Recently, it has become apparent that a large, highly diverse group of proteins including several transcription factors and other DNA-interacting proteins, contain one or more regions similar to HMG1, and this feature has come to be known as the HMG1 box or HMG1 domain. cDNAs coding for HMG1 have been cloned from human, rat, mouse, mole rat, trout, hamster, pig and calf cells, and HMG1 is believed to be abundant in all vertebrate cell nuclei. The protein is highly conserved with interspecies sequence identities in the 80% range. In chromatin, HMG1 binds to linker DNA between nucleosomes and to a variety of non-β-DNA structures such as palindromes, cruciforms and stem-loop structures, as well as cisplatin-modified DNA. DNA binding by HMG1 is generally believed to be sequence insensitive. HMG1 is most frequently prepared from washed nuclei or chromatin, but the protein has also been detected in the cytoplasm. (Reviewed in Landsman and Bustin, BioEssays 15:539-546, 1993; Baxevanis and Landsman, Nucleic Acids Research 23:514-523, 1995).

The present invention also relates to variants, derivatives and fragments of HMGB1. Preferably, the variant sequences etc. are at least as biologically active as the sequences presented herein.

As used herein "biologically active" refers to a sequence having a similar structural function (but not necessarily to the same degree), and/or similar regulatory function (but not necessarily to the same degree), and/or similar biochemical function (but not necessarily to the same degree) of the naturally occurring sequence.

Preferably such variants, derivative and fragments comprise one or both the HMG boxes.

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10 or 20 amino acids.

It will be understood that amino acid sequences for use in the invention are not limited to the particular sequences or fragments thereof or sequences obtained from a particular protein but also include homologous sequences obtained from any source, for example related viral/bacterial proteins, cellular homologues and synthetic peptides, as well as variants or derivatives thereof.

Thus, the present invention covers variants, homologues or derivatives of the amino acid sequences for use in the present invention, as well as variants, homologues or derivatives of the nucleotide sequence coding for the amino acid sequences used in the present invention.

In the context of the present invention, a homologous sequence is taken to include an amino acid sequence which is at least 60, 70, 80 or 90% identical, preferably at least 95 or 98% identical at the amino acid level. In particular, homology should typically be considered with respect to those regions of the sequence known to be essential for APC activation rather than non-essential neighbouring sequences. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al*., 1999 *ibid* - Chapter 18), FASTA (Atschul et al., 1990, J. Mol. Biol., 403-410) and the GENEWORKS suite of comparison tools. Both BLAST and FASTA are available for offline and online searching (see Ausubel *et al*., 1999 *ibid*, pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

### HMGB1 Variants and Derivatives

The terms "variant" or "derivative" in relation to the amino acid sequences of the present invention includes any substitution of, variation of, modification of, replacement of, deletion of or addition of one (or more) amino acids from or to the sequence providing the resultant amino acid sequence has APC activation activity, preferably having at least the same activity as human HMGB1.

HMGB1 may be modified for use in the present invention. Typically, modifications are made that maintain the activity of the sequence. Amino acid substitutions may be made, for example from 1, 2 or 3 to 10, 20 or 30 substitutions provided that the modified sequence retains the APC activation activity. Amino acid substitutions may include the use of non-naturally occurring analogues, for example to increase blood plasma half-life of a therapeutically administered polypeptide.

Conservative substitutions may be made, for example according to the Table below. Amino acids in the same block in the second column and preferably in the same line in the third column may be substituted for each other:

| | | |
|---|---|---|
| ALIPHATIC | Non-polar | GAP |
| | | ILV |
| | Polar - uncharged | C S T M |
| | | NQ |
| | Polar - charged | D E |
| | | KR |
| AROMATIC | | H F W Y |

Proteins for use in the invention are typically made by recombinant means, for example as described below. However they may also be made by synthetic means using techniques well known to skilled persons such as solid phase synthesis. Proteins for use in the invention may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the activity of the protein of interest.

Proteins for use in the invention may be in a substantially isolated form. It will be understood that the protein may be mixed with carriers or diluents which will not interfere with the intended purpose of the protein and still be regarded as substantially isolated. A protein of the invention may also be in a substantially purified form, in which case it will generally comprise the protein in a preparation in which more than 90%, e.g. 95%, 98% or 99% of the protein in the preparation is a protein of the invention.

### Polynucleotides

Polynucleotides for use in the disclosure comprise nucleic acid sequences encoding the HMGB1 proteins, including derivatives, variants, fragments etc., for use in the invention. It will be understood by a skilled person that numerous different polynucleotides can encode the same protein as a result of the degeneracy of the genetic code. In addition, it is to be understood that skilled persons may, using routine techniques, make nucleotide substitutions that do not affect the protein sequence encoded by the polynucleotides of the invention to reflect the codon usage of any particular host organism in which the proteins for use in the invention are to be expressed.

Polynucleotides for use in the disclosure may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of polynucleotides for use in the invention.

The terms "variant", "homologue" or "derivative" in relation to the nucleotide sequence include any substitution of, variation of, modification of, replacement of, deletion of or addition of one (or more) nucleic acid from or to the sequence providing the resultant nucleotide sequence codes for a polypeptide having the capability to activate APCs..

As indicated above, with respect to sequence homology, preferably there is at least 75%, more preferably at least 85%, more preferably at least 90% homology to the sequences shown in the sequence listing herein. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

The disclosure also encompasses nucleotide sequences that are capable of hybridising selectively to the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides for use in the disclosure capable of selectively hybridising to the nucleotide sequences presented herein, or to their complement, will be generally at least 70%, preferably at least 80 or 90% and more preferably at least 95% or 98% homologous to the corresponding nucleotide sequences presented herein over a region of at least 20, preferably at least 25 or 30, for instance at least 40, 60 or 100 or more contiguous nucleotides. Preferred polynucleotides for use in the invention will comprise regions homologous to the HMG box, preferably at least 80 or 90% and more preferably at least 95% homologous to the HMG box.

The term "selectively hybridizable" means that the polynucleotide used as a probe is used under conditions where a target polynucleotide for use in the invention is found to hybridize to the probe at a level significantly above background. The background hybridization may occur because of other polynucleotides present, for example, in the cDNA or genomic DNA library being screening. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with ³²P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

In a preferred aspect, the present invention covers nucleotide sequences that can hybridise to the nucleotide sequence of the present invention under stringent conditions (e.g. 65°C and 0.1xSSC {1xSSC = 0.15 M NaCl, 0.015 M Na₃ Citrate pH 7.0).

Where the polynucleotide for use in the disclosure is double-stranded, both strands of the duplex, either individually or in combination, are encompassed by the present invention. Where the polynucleotide is single-stranded, it is to be understood that the complementary sequence of that polynucleotide is also included within the scope of the present invention.

Polynucleotides which are not 100% homologous to the sequences used in the present invention but fall within the scope of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of the human HMGB1 sequence under conditions of medium to high stringency. Similar considerations apply to obtaining species homologues and allelic variants of the protein or nucleotide sequences for use in the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis. This may be useful where for example silent codon changes are required to sequences to optimise codon preferences for a particular host cell in which the polynucleotide sequences are being expressed. Other sequence changes may be desired in order to introduce restriction enzyme recognition sites.

Polynucleotides of the disclosure may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as a DNA polynucleotides and probes for use in the disclosure may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

### Nucleotide vectors

Polynucleotides of the disclosure can be incorporated into a recombinant replicable vector. The vector may be used to replicate the nucleic acid in a compatible host cell. Thus in a further embodiment, the invention provides a method of making polynucleotides for use in the invention by introducing a polynucleotide of the invention into a replicable vector, introducing the vector into a compatible host cell, and growing the host cell under conditions which bring about replication of the vector. The vector may be recovered from the host cell. Suitable host cells include bacteria such as *E. coli*, yeast, mammalian cell lines and other eukaryotic cell lines, for example insect Sf9 cells.

Preferably, a polynucleotide of the disclosure in a vector is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under condition compatible with the control sequences.

The control sequences may be modified, for example by the addition of further transcriptional regulatory elements to make the level of transcription directed by the control sequences more responsive to transcriptional modulators.

Vectors of the disclosure may be transformed or transfected into a suitable host cell as described below to provide for expression of a protein of the invention. This process may comprise culturing a host cell transformed with an expression vector as described above under conditions to provide for expression by the vector of a coding sequence encoding the protein, and optionally recovering the expressed protein.

The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used, for example, to transfect or transform a host cell.

In preferred embodiments HMGB1 may be produced by bacterial cells (Bianchi 1991, Gene 104: 271-275; Lee et al. 1998, Gene 225: 97-105), by yeasts (Mistry et al. 1997, Biotechniques 22: 718-729), or by purification from cell cultures or from mammalian tissues.

Vectors/polynucleotides for use in the disclosure may introduced into suitable host cells using a variety of techniques known in the art, such as transfection, transformation and electroporation. Where vectors/polynucleotides of the disclosure are to be administered to animals, several techniques are known in the art, for example infection with recombinant viral vectors such as retroviruses, herpes simplex viruses and adenoviruses, direct injection of nucleic acids and biolistic transformation.

### Protein Expression and Purification

Host cells comprising polynucleotides of the disclosure may be used to express proteins for use in the invention. Host cells may be cultured under suitable conditions which allow expression of the proteins of the invention. Expression of the proteins of the disclosure may be constitutive such that they are continually produced, or inducible, requiring a stimulus to initiate expression. In the case of inducible expression, protein production can be initiated when required by, for example, addition of an inducer substance to the culture medium, for example dexamethasone or IPTG.

Proteins for use in the invention can be extracted from host cells by a variety of techniques known in the art, including enzymatic, chemical and/or osmotic lysis and physical disruption.

### Immune reponse

The present invention relates to a method of modulating an immune response, and in particular an antigen-mediated immune response.

In addition to triggering non-specific mechanisms, pathogens, e.g. during an infection, also trigger the antigen-specific adaptive immune response. The adaptive immune response to infection involves both the T and B cell mediated compartments of the immune system. The present invention relates particularly to the so-called induction phase during which antigen presenting cells (APCs) are involved in the initiation of the adaptive immune response. APC function is also required for maintenance of the adaptive immune response.

In more detail, APCs constitute a complex of cells capable of internalizing an antigen, processing it and expressing epitopes thereof in association with class I and class II MHC molecules. In general it can be said that the common characteristic of the cells of the group of APCs used medically is the expression of MHC molecules of class II as well as class I on the cell surface. The group mainly comprises dendritic cells, activated macrophages, microglial cells of the central nervous system and B lymphocytes. Among these, the dendritic cells (DCs) are particularly specialized in antigen presentation and constitute a population with distinctive characteristics and are widely distributed in tissues. The DCs are involved in the activation of the immune response, which takes place by stimulation of the T lymphocytes in the course of various pathologies such as infections, autoimmune diseases and transplant rejection. Activation or maturation of DCs is a necessary process for "priming" the T cells and initiating the immune response.

In autoimmune diseases and in transplant rejection, i.e. in the absence of pathogenic agents, induction of maturation of the dendritic cells takes place by means of endogenous molecules possessing immunostimulatory activity *in vivo.* It is known that cells that are dying contain and release molecules that are able to amplify the immune response (3-8). These molecules, normally segregated inside living cells, remain thus in the apoptotic process while they are released during cell death.

Cellular constituents released in the culture medium after cell death are able to provoke maturation of the DCs (3, 4). On the other hand, DCs stimulated with cells in the initial apoptotic state, or with their culture medium, are not activated (3-5, 9). Similarly, the DCs are not activated by necrotic polymorphonuclear (PMN) leukocytes.

We have now found that HMGB1 is capable of activating the maturation of APCs. By "activating" we include inducing maturation of APCs. Conversely we have found that an antagonist of HMGB1 is capable of preventing or reducing the activation of an APC. Thus, for example, when an antagonist of HMGB1 is added to a population of APCs in conditions in which maturation is capable of occurring, fewer APCs proceed to maturity than in the absence of the HMGB1 antagonist.

### APCs

Antigen presenting cells (APCs) include macrophages, dentritic cells, B cells and virtually any other cell type capable of expressing an MHC molecule.

Macrophages are phagocytic cells of the monocytic lineage residing within tissues and are particularly well equipped for effective antigen presentation. They generally express MHC class II molecules and along with their phagocytic properties are extremely efficient at engulfing macromolecular or particulate material, digesting it, processing it with an extensive lysosomal system to antigenic peptide form, and expressing it on the cell surface for recognition by T lymphocytes.

Dendritic cells, so named for their highly branched morphology, are found in many organs throughout the body, are bone marrow-derived and usually express high levels of MHC class II antigen. Dendritic cells are actively motile and can recirculate between the bloodstream and tissues. In this way, they are considered the most important APCs. Langerhans cells are an example of dendritic cells that are located in the skin.

B lymphocytes, while not actively phagocytic, are class II-positive and possess cell surface antigen-specific receptors, immunoglobulin, or antibody molecules. Due to their potential for high affinity antigen binding, B cells are uniquely endowed with the capacity to concentrate low concentrations of antigen on their surface, endocytose it, process it and present it in the context of antigenic peptide in association with MHC antigen on their surface. In this manner, B cells become extremely effective APCs.

### Vaccine

Another aspect of the invention relates to a method for inducing an immunological response in an individual, particularly a mammal, preferably humans, which comprises inoculating the individual with the HMGB1 protein of the present invention, or a fragment or variant thereof, adequate to produce antibody and/ or T cell immune response to protect said individual from for example a tumor or infection such as a bacterial or viral infection. Also provided are methods whereby such immunological response slows tumor growth or viral or bacterial replication.

A further aspect of the invention relates to an immunological composition that when introduced into an individual, preferably a human, capable of having induced within it an immunological response, induces an immunological response in such individual. The immunological response may be used therapeutically or prophylactically and may take the form of antibody immunity and/or cellular immunity, such as cellular immunity arising from CTL or CD4+ T cells.

The immunological response may be to a HMGB1 protein of the present invention; however we have surprisingly found that the HMGB1 protein may be used as an adjuvant in a composition wherein the immunological response is directed to another antigen. Thus, HMGB 1 may be used as an adjuvant in a vaccine composition.

The preparation of vaccines which contain an immunogenic polypeptide(s) as active ingredient(s), is known to one skilled in the art. Typically, such vaccines are prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof.

In addition, if desired, the vaccine may contain minor amounts of auxiliary substances such as wetting or emulsifying agents, pH buffering agents, and/or adjuvants which enhance the effectiveness of the vaccine.

The vaccine formulation of the invention preferably relates to and/or includes an adjuvant system for enhancing the immunogenicity of the formulation. Preferably the adjuvant system raises predominantly a TH1 type of response.

An immune response may be broadly distinguished into two extreme categories, being a humoral or cell mediated immune responses (traditionally characterised by antibody and cellular effector mechanisms of protection respectively). These categories of response have been termed TH1-type responses (particularly efficient against intracellular pathogens and tumor cells), and TH2-type immune responses (humoral response, mainly involved in the response to extracellular pathogens).

Extreme TH1-type immune responses may be characterised by the generation of antigen specific, haplotype restricted cytotoxic T lymphocytes, and natural killer cell responses. In mice TH1-type responses are often characterised by the generation of antibodies of the IgG2a subtype, whilst in the human these correspond to IgG1 type antibodies. TH2-type immune responses are characterised by the generation of a broad range of immunoglobulin isotypes including in mice IgG1, IgA, and IgM.

It can be considered that the driving force behind the development of these two types of immune responses are cytokines. High levels of TH1-type cytokines tend to favour the induction of cell mediated immune responses to the given antigen, whilst high levels of TH2-type cytokines tend to favour the induction of humoral immune responses to the antigen.

The distinction of TH1 and TH2-type immune responses is not absolute. In reality an individual will support an immune response which is described as being predominantly TH1 or predominantly TH2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, TH1-type responses are associated with the production of the INF-γ and IL-2 cytokines by T-lymphocytes. Other cytokines often directly associated with the induction of TH1-type immune responses are not produced by T-cells, such as IL-12. In contrast, TH2- type responses are associated with the secretion of IL-4, IL-5, IL-6 and IL-13.

It is known that certain vaccine adjuvants are particularly suited to the stimulation of either TH1 or TH2 - type cytokine responses. Traditionally the best indicators of the TH1:TH2 balance of the immune response after a vaccination or infection includes direct measurement of the production of TH1 or TH2 cytokines by T lymphocytes *in vitro* after restimulation with antigen, and/or the measurement of the IgG1:IgG2a ratio of antigen specific antibody responses.

Thus, a TH1-type adjuvant is one which preferentially stimulates isolated T-cell populations to produce high levels of TH1-type cytokines when re-stimulated with antigen *in vitro,* and promotes development of both CD8+ cytotoxic T lymphocytes and antigen specific immunoglobulin responses associated with TH1-type isotype.

The HMGB1 protein of the present invention may be used as an adjuvant in a variety of vaccine types. Non-limiting examples of such vaccine types include subunit vaccines and cellular vaccines, e.g. immunotherapy of tumors with dendritic cells.

The composition of the present invention may include (additional) adjuvants. Examples of adjuvants and other agents include aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, *Corynebacterium parvum (Propionobacterium acnes), Bordetella pertussis,* polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

Typically, adjuvants such as Amphigen (oil-in-water), Alhydrogel (aluminum hydroxide), or a mixture of Amphigen and Alhydrogel are used. Only aluminum hydroxide is approved for human use.

The proportion of immunogen and adjuvant can be varied over a broad range so long as both are present in effective amounts. For example, aluminum hydroxide can be present in an amount of about 0.5% of the vaccine mixture (Al₂O₃ basis). Conveniently, the vaccines are formulated to contain a final concentration of immunogen in the range of from 0.2 to 200 µg/ml, preferably 5 to 50 µg/ml, most preferably 15 µg/ml.

After formulation, the vaccine may be incorporated into a sterile container which is then sealed and stored at a low temperature, for example 4°C, or it may be freeze-dried. Lyophilisation permits long-term storage in a stabilised form.

The effectiveness of an adjuvant may be determined by measuring the amount of antibodies or T cells directed against an immunogenic polypeptide containing an antigenic sequence resulting from administration of this polypeptide in vaccines which are also comprised of the various adjuvants.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

The polypeptides of the invention may be formulated into the vaccine as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric and maleic. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

Additional vaccinations have been recently developed that rely on the injection of APCs either subcutaneously, intradermis, intravenously, intranodally, or intra-tumour. Cells are usually resuspended in appropriate isotonic media before injection, which may be further supplemented by adjuvants.

### Agonists and Antagonists

The methods and compositions of our invention rely, in some embodiments, on blocking the activity HMGB1. It is also possible in other embodiments to use agents which upregulate HMGB1. Agents which are capable of increasing the activity of HMGB1 are referred to as agonists of that activity. Similarly, antagonists reduce the activity of the HMGB1.

The term "antagonist", as used in the art, is generally taken to refer to a compound which binds to an enzyme and inhibits the activity of the enzyme. The term as used here, however, is intended to refer broadly to any agent which inhibits the activity of a molecule, not necessarily by binding to it.

Accordingly, it includes agents which affect the expression of a protein, or the biosynthesis of a molecule, or the expression of modulators of the activity of the inhibitor. The specific activity which is inhibited may be any activity which is characteristic of the molecule, for example, the ability to activate APCs. Assays for APC activation are known in the art.

The antagonist may bind to and compete for one or more sites on the relevant molecule, for example, the HMG box. Preferably, such binding blocks the interaction between the molecule and another entity

Blocking the activity of an HMGB1 protein or protein inhibitor may also be achieved by reducing the level of expression of the protein or inhibitor in the cell. For example, the cell may be treated with antisense compounds, for example oligonucleotides having sequences specific to the protein or protein inhibitor mRNA.

As used herein, in general, the term "antagonist" includes but is not limited to agents such as an atom or molecule, wherein a molecule may be inorganic or organic, a biological effector molecule and/or a nucleic acid encoding an agent such as a biological effector molecule, a protein, a polypeptide, a peptide, a nucleic acid, a peptide nucleic acid (PNA), a virus, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a fatty acid and a carbohydrate. An agent may be in solution or in suspension (e.g., in crystalline, colloidal or other particulate form). The agent may be in the form of a monomer, dimer, oligomer, etc, or otherwise in a complex.

The terms "antagonist" and "agent" are also intended to include, a protein, polypeptide or peptide including, but not limited to, a structural protein, an enzyme, a cytokine (such as an interferon and/or an interleukin) an antibiotic, a polyclonal or monoclonal antibody, or an effective part thereof, such as an Fv fragment, which antibody or part thereof may be natural, synthetic or humanised, a peptide hormone, a receptor, a signalling molecule or other protein; a nucleic acid, as defined below, including, but not limited to, an oligonucleotide or modified oligonucleotide, an antisense oligonucleotide or modified antisense oligonucleotide, cDNA, genomic DNA, an artificial or natural chromosome (e.g. a yeast artificial chromosome) or a part thereof, RNA, including mRNA, tRNA, rRNA or a ribozyme, or a peptide nucleic acid (PNA); a virus or virus-like particles; a nucleotide or ribonucleotide or synthetic analogue thereof, which may be modified or unmodified; an amino acid or analogue thereof, which may be modified or unmodified; a non-peptide (e.g., steroid) hormone; a proteoglycan; a lipid; or a carbohydrate. Small molecules, including inorganic and organic chemicals, which bind to and occupy the active site of the polypeptide thereby making the catalytic site inaccessible to substrate such that normal biological activity is prevented, are also included. Examples of small molecules include but are not limited to small peptides or peptide-like molecules.

### Antisense Compounds

As described above, the antagonist may comprise one or more antisense compounds, including antisense RNA and antisense DNA, which are capable of reducing the level of expression of the HMGB1. Preferably, the antisense compounds comprise sequences complementary to the mRNA encoding the HMGB 1.

Preferably, the antisense compounds are oligomeric antisense compounds, particularly oligonucleotides. The antisense compounds preferably specifically hybridize with one or more nucleic acids encoding the HMGB1. As used herein, the term "nucleic acid encoding HMGB1 encompasses DNA encoding the HMGB1, RNA (including pre-mRNA and mRNA) transcribed from such DNA, and also cDNA derived from such RNA. The specific hybridization of an oligomeric compound with its target nucleic acid interferes with the normal function of the nucleic acid. This modulation of function of a target nucleic acid by compounds which specifically hybridize to it is generally referred to as "antisense". The functions of DNA to be interfered with include replication and transcription. The functions of RNA to be interfered with include all vital functions such as, for example, translocation of the RNA to the site of protein translation, translation of protein from the RNA, splicing of the RNA to yield one or more mRNA species, and catalytic activity which may be engaged in or facilitated by the RNA. The overall effect of such interference with target nucleic acid function is modulation of the expression of the HMGB1. In the context of the present invention, "modulation" means either an increase (stimulation) or a decrease (inhibition) in the expression of a gene. For example, the expression of a gene encoding an inhibitor of HMGB1 activity, or an inhibitor of expression of the HMGB1 may be increased. However, preferably, inhibition of expression, in particular, inhibition of HMGB1 expression, is the preferred form of modulation of gene expression and mRNA is a preferred target.

Antisense constructs are described in detail in US 6,100,090 (Monia et al), and Neckers et al., 1992, Crit Rev Oncog 3(1-2):175-231.

### Antibodies

The disclosure also provides monoclonal or polyclonal antibodies to proteins for use disclose herein or fragments thereof. Thus, the disclosure further provides a process for the production of monoclonal or polyclonal antibodies to proteins for use disclosed herein.

If polyclonal antibodies are desired, a selected mammal (e.g., mouse, rabbit, goat, horse, etc.) is immunised with an immunogenic polypeptide bearing an HMGB1 epitope(s). Serum from the immunised animal is collected and treated according to known procedures. If serum containing polyclonal antibodies to an epitope contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against epitopes in the polypeptides of the invention can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against epitopes can be screened for various properties; i.e., for isotype and epitope affinity.

An alternative technique involves screening phage display libraries where, for example the phage express scFv fragments on the surface of their coat with a large variety of complementarity determining regions (CDRs). This technique is well known in the art.

Antibodies, both monoclonal and polyclonal, which are directed epitopes are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the antigen of the agent against which protection is desired.

Techniques for raising anti-idiotype antibodies are known in the art. These anti-idiotype antibodies may also be useful in therapy.

For the purposes of this invention, the term "antibody", unless specified to the contrary, includes fragments of whole antibodies which retain their binding activity for a target antigen. Such fragments include Fv, F(ab') and F(ab')₂ fragments, as well as single chain antibodies (scFv). Furthermore, the antibodies and fragments thereof may be humanised antibodies, for example as described in EP-A-239400.

Antibodies may be used in method of detecting polypeptides of the disclosure present in biological samples by a method which comprises:
(a) providing an antibody of the disclosure;
(b) incubating a biological sample with said antibody under conditions which allow for the formation of an antibody-antigen complex; and
(c) determining whether an antibody-antigen complex comprising said antibody is formed.

Suitable samples include extracts from tissues such as brain, breast, ovary, lung, colon, pancreas, testes, liver, muscle and bone tissues or from neoplastic growths derived from such tissues.

Antibodies of the invention may be bound to a solid support and/or packaged into kits in a suitable container along with suitable reagents, controls, instructions and the like.

### Assays

The disclosure also provides a method of screening compounds to identify agonists and antagonists to HMGB1. Candidate compounds may be identified from a variety of sources, for example, cells, cell-free preparations, chemical libraries, peptide and gene libraries, and natural product mixtures. Such agonists or antagonists or inhibitors so-identified may be natural or modified substrates, ligands, receptors, enzymes, etc., as the case may be, of the retinol binding protein receptor; or may be structural or functional mimetics thereof (see Coligan et al., Current Protocols in Immunology 1(2):Chapter 5 (1991)).

The screening method may simply measure the binding of a candidate compound to HMGB1 by means of a label directly or indirectly associated with the candidate compound. Alternatively, the screening method may involve competition with a labeled competitor. Further, these screening methods may test whether the candidate compound results in a signal generated by activation or inhibition of HMGB1, using detection systems appropriate to the cells bearing the receptor. A compound which binds but does not elicit a response identifies that compound as an antagonist. An antagonist compound is also one which binds and produces an opposite response, in other words, reduction of proliferation and optionally induction of differentiation.

### Therapeutic proteins

Proteins of the disclosure may be administered therapeutically to patients. It is preferred to use proteins that do not consisting solely of naturally-occurring amino acids but which have been modified, for example to reduce immunogenicity, to increase circulatory half-life in the body of the patient, to enhance bioavailability and/or to enhance efficacy and/or specificity.

A number of approaches have been used to modify proteins for therapeutic application. One approach is to link the peptides or proteins to a variety of polymers, such as polyethylene glycol (PEG) and polypropylene glycol (PPG) - see for example U.S. Patent Nos. 5,091,176, 5,214,131 and US 5,264,209.

Replacement of naturally-occurring amino acids with a variety of uncoded or modified amino acids such as D-amino acids and N-methyl amino acids may also be used to modify proteins

Another approach is to use bifunctional crosslinkers, such as N-succinimidyl 3-(2 pyridyldithio) propionate, succinimidyl 6-[3-(2 pyridyldithio) propionamido] hexanoate, and sulfosuccinimidyl 6-[3-(2 pyridyldithio) propionamido]hexanoate (see US Patent 5,580,853).

It may be desirable to use derivatives of the proteins of the disclosure which are conformationally constrained. Conformational constraint refers to the stability and preferred conformation of the three-dimensional shape assumed by a protein. Conformational constraints include local constraints, involving restricting the conformational mobility of a single residue in a protein; regional constraints, involving restricting the conformational mobility of a group of residues, which residues may form some secondary structural unit; and global constraints, involving the entire protein structure.

The active conformation of the protein may be stabilised by a covalent modification, such as cyclization or by incorporation of gamma-lactam or other types of bridges. For example, side chains can be cyclized to the backbone so as create a L-gamma-lactam moiety on each side of the interaction site. See, generally, Hruby et al., "Applications of Synthetic Peptides," in Synthetic Peptides: A User's Guide: 259-345 (W. H. Freeman & Co. 1992). Cyclization also can be achieved, for example, by formation of cysteine bridges, coupling of amino and carboxy terminal groups of respective terminal amino acids, or coupling of the amino group of a Lys residue or a related homolog with a carboxy group of Asp, Glu or a related homolog. Coupling of the .alpha-amino group of a polypeptide with the epsilon-amino group of a lysine residue, using iodoacetic anhydride, can be also undertaken. See Wood and Wetzel, 1992, Int'l J. Peptide Protein Res. 39: 533-39.

Another approach described in US 5,891,418 is to include a metal-ion complexing backbone in the protein structure. Typically, the preferred metal-peptide backbone is based on the requisite number of particular coordinating groups required by the coordination sphere of a given complexing metal ion. In general, most of the metal ions that may prove useful have a coordination number of four to six. The nature of the coordinating groups in the protein chain includes nitrogen atoms with amine, amide, imidazole, or guanidino functionalities; sulfur atoms of thiols or disulfides; and oxygen atoms of hydroxy, phenolic, carbonyl, or carboxyl functionalities. In addition, the protein chain or individual amino acids can be chemically altered to include a coordinating group, such as for example oxime, hydrazino, sulfhydryl, phosphate, cyano, pyridino, piperidino, or morpholino. The protein construct can be either linear or cyclic, however a linear construct is typically preferred. One example of a small linear peptide is Gly-Gly-Gly-Gly which has four nitrogens (an N₄ complexation system) in the back bone that can complex to a metal ion with a coordination number of four.

A further technique for improving the properties of therapeutic proteins is to use non-peptide peptidomimetics. A wide variety of useful techniques may be used to elucidating the precise structure of a protein. These techniques include amino acid sequencing, x-ray crystallography, mass spectroscopy, nuclear magnetic resonance spectroscopy, computer-assisted molecular modelling, peptide mapping, and combinations thereof. Structural analysis of a protein generally provides a large body of data which comprise the amino acid sequence of the protein as well as the three-dimensional positioning of its atomic components. From this information, non-peptide peptidomimetics may be designed that have the required chemical functionalities for therapeutic activity but are more stable, for example less susceptible to biological degradation. An example of this approach is provided in US 5,811,512.

Techniques for chemically synthesising therapeutic proteins of the invention are described in the above references and also reviewed by Borgia and Fields, 2000, TibTech 18: 243-251 and described in detail in the references contained therein.

### Methods

Immature dendritic cells for use in the present invention can be obtained from haematopoietic precursors or from stem cells, for example from PBMC cells, by suitable treatment with cytokines such as GM-CSF, IL-4 and flt3-L.

The activation or maturation of antigen-presenting cells can be effected starting from a culture of immature or inactive cells, by adding HMGB1 protein and possibly other co-adjuvants such as cytokines to the culture medium.

Once led to maturation or activated, the antigen-presenting cells, especially the DCs, can be used for the activation of T lymphocytes in response to particular antigens; the lymphocytes thus activated can then be administered to a subject to stimulate their immune response to the said antigens.

The indicators of activation can vary according to the cell type under consideration. With regard to macrophages, microglia and B lymphocytes, for example, it is a functional activation with increase in membrane expression of MHC molecules and co-stimulatory molecules following contact with other adjuvants, as described in (27, 28).

In the case of the dendritic cells, those cells that display increased expression of markers characteristic of the "maturation phenotype", such as the CD83 and CD86 surface molecules, or reduced expression of markers characteristic of the immature phenotype, such as CD115, CD14, CD68 and CD32, are regarded as activated or mature.

According to a one embodiment, the invention therefore relates to an *ex vivo* method for the activation of T lymphocytes that comprises the following steps:
a) bringing a preparation of inactive APCs into contact with HMGB1, or with its biologically active fragments, so as to induce their activation;
b) bringing the activated APCs into contact with a particular antigen;
c) exposing the T lymphocytes to the APCs that have been activated and exposed to the antigen.

According to a preferred embodiment, dendritic cells are used as APCs.

Steps a) - c) indicated above can be executed in a different order. For example, the antigen can be added to a culture of immature or inactive APCs before the HMGB1 protein or its fragments. In addition, the APCs or DCs can be transfected with a vector for the expression of a particular antigen or of a polypeptide derived from it, or alternatively a vector for the expression of a specific MHC molecule. Antigens associated with microorganisms, viruses, tumours or autoimmune diseases can be used for the activation of lymphocytes according to the method described. As tumour antigens, in addition to the proteins or their fragments isolated from tumour tissues or cells, it is possible to use whole cells that have been killed by apoptosis or necrosis. It is also possible to use antigens associated with viruses or retroviruses, especially HIV, or with intracellular pathogens, such as mycobacteria or plasmodia.

In another embodiment the present invention relates to an *in vivo* method in which HMGB1 and optionally an antigen are introduced into a patient, for example into a lymph node or into a tumour. The antigen may be introduced before, at the same time as, or after the HMGB 1. Alternatively the antigen may be present *in vivo,* for example as an HLA antigen or have been introduced during a transplant.

In all embodiments the HMGB1 and/or antigen may be introduced as a polynucleotide sequence, i.e. using a gene delivery approach.

### Introduction of nucleic acid sequences into APCs

APCs as described above may be cultured in a suitable culture medium such as DMEM or other defined media, optionally in the presence of fetal calf serum.

HMGB1 may be administered to APCs and by introducing nucleic acid constructs/viral vectors encoding the protein into cells under conditions that allow for expression of the polypeptide in the APC. Similarly, nucleic acid constructs encoding antisense constructs may be introduced into the APCs and by transfection, viral infection or viral transduction.

The antagonist of the present invention may also be administered in a similar way to HMGB1.

### Delivery Systems

The disclosure further provides a delivery system for a protein, polynucelotide, agonist or antagonist of the disclosure. For ease of reference to protein, agonist and/or antagonist will be referred to as "agent" in the present section.

The delivery system of the present disclosure may be a viral or non-viral delivery system. Non-viral delivery mechanisms include but are not limited to lipid mediated transfection, liposomes, immunoliposomes, lipofectin, cationic facial amphiphiles (CFAs) and combinations thereof. As previously indicated when the agent is delivered in the form of a polynucleotide to a cell for subsequent expression therein the agent is preferably delivered via a retroviral vector delivery system. However, the polynucleotide may be delivered to the target cell population by any suitable Gene Delivery Vehicle, GDV. This includes but is not restricted to, DNA, formulated in lipid or protein complexes or administered as naked DNA via injection or biolistic delivery, and viruses such as retroviruses. Alternatively, the polynucleotides are delivered by cells such as monocytes, macrophages, lymphocytes or hematopoietic stem cells. In particular a cell-dependent delivery system is used. In this system the polynucleotides encoding the agent are introduced into one or more cells *ex vivo* and then introduced into the patient.

The agents of the present invention may be administered alone but will generally be administered as a pharmaceutical composition.

### Treatment

This includes any therapeutic application that can benefit a human or non-human animal. The treatment of mammals is particularly preferred. Both human and veterinary treatments are within the scope of the present disclosure.

Treatment may be in respect of an existing condition or it may be prophylactic. It may be of an adult, a juvenile, an infant, a foetus, or a part of any of the aforesaid (e.g. an organ, tissue, cell, or nucleic acid molecule).

The APCs prepared by the method of the disclosure may be administered to a patient suffering from a malignancy.

Generally, in an *ex vivo* approach the patient will be the same patient from whom the treated APCs originated. Examples of malignancies that may be treated include cancer of the breast, cervix, colon, rectum, endometrium, kidney, lung, ovary, pancreas, prostate gland, skin, stomach, bladder, CNS, oesophagus, head-or-neck, liver, testis, thymus or thyroid. Malignancies of blood cells, bone marrow cells, B-lymphocytes, T-lymphocytes, lymphocytic progenitors or myeloid cell progenitors may also be treated.

The tumour may be a solid tumour or a non-solid tumour and may be a primary tumour or a disseminated metastatic (secondary) tumour. Non-solid tumours include myeloma; leukaemia (acute or chronic, lymphocytic or myelocytic) such as acute myeloblastic, acute promyelocytic, acute myelomonocytic, acute monocytic, erythroleukaemia; and lymphomas such as Hodgkin's, non-Hodgkin's and Burkitt's. Solid tumours include carcinoma, colon carcinoma, small cell lung carcinoma, non-small cell lung carcinoma, adenocarcinoma, melanoma, basal or squamous cell carcinoma, mesothelioma, adenocarcinoma, neuroblastoma, glioma, astrocytoma, medulloblastoma, retinoblastoma, sarcoma, osteosarcoma, rhabdomyosarcoma, fibrosarcoma, osteogenic sarcoma, hepatoma, and seminoma.

Typically the composition of the present disclosure may be administered with a tumour-specific antigen such as antigens which are overexpressed on the surface of tumour cells.

The APCs may be used to treat an ongoing immune response (such as an allergic condition or an autoimmune disease) or may be used to generate tolerance in a patient. Thus the cells of the present invention may be used in therapeutic methods for both treating and preventing diseases characterised by inappropriate lymphocyte activity in animals and humans. The APCs may be used to confer tolerance to a single antigen or to multiple antigens.

Typically, APCs are obtained from the patient or donor and primed as described above before being returned to the patient (*ex vivo* therapy).

Particular conditions that may be treated or prevented include multiple sclerosis, rheumatoid arthritis, diabetes, allergies, asthma, and graft rejection. The present invention may also be used in organ transplantation or bone marrow transplantation.

### Pharmaceutical compositions

A pharmaceutical composition is a composition that comprises or consists of a therapeutically effective amount of a pharmaceutically active agent. It preferably includes a pharmaceutically acceptable carrier, diluent or excipients (including combinations thereof). Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

"Therapeutically effective amount" refers to the amount of the therapeutic agent which is effective to achieve its intended purpose. While individual patient needs may vary, determination of optimal ranges for effective amounts of HMGB1 is within the skill of the art. Generally the dosage regimen for treating a condition with the compounds and/or compositions of this invention is selected in accordance with a variety of factors, including the type, age, weight, sex, diet and medical condition of the patient, the severity of the dysfunction, the route of administration, pharmacological considerations such as the activity, efficacy, pharmacokinetic and toxicology profiles of the particular compound used, whether a drug delivery system is used, and whether the compound is administered as part of a drug combination and can be adjusted by one skilled in the art. Thus, the dosage regimen actually employed may vary widely and therefore may deviate from the preferred dosage regimen set forth herein.

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Typically, each conjugate may be administered at a dose of from 0.01 to 30 mg/kg body weight, preferably from 0.1 to 10 mg/kg, more preferably from 0.1 to 1 mg/kg body weight.

When the polynucleotides/vectors are administered as a naked nucleic acid, the amount of nucleic acid administered may typically be in the range of from 1 µg to 10 mg, preferably from 100 µg to 1 mg.

Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam^{™} and transfectam^{™}). Typically, nucleic acid constructs are mixed with the transfection agent to produce a composition.

The routes of administration and dosages described are intended only as a guide since a skilled practitioner will be able to determine readily the optimum route of administration and dosage for any particular patient and condition.

### Description of the Figures

The present invention will now be described further with reference to the following non-limiting Examples and Figures in which:

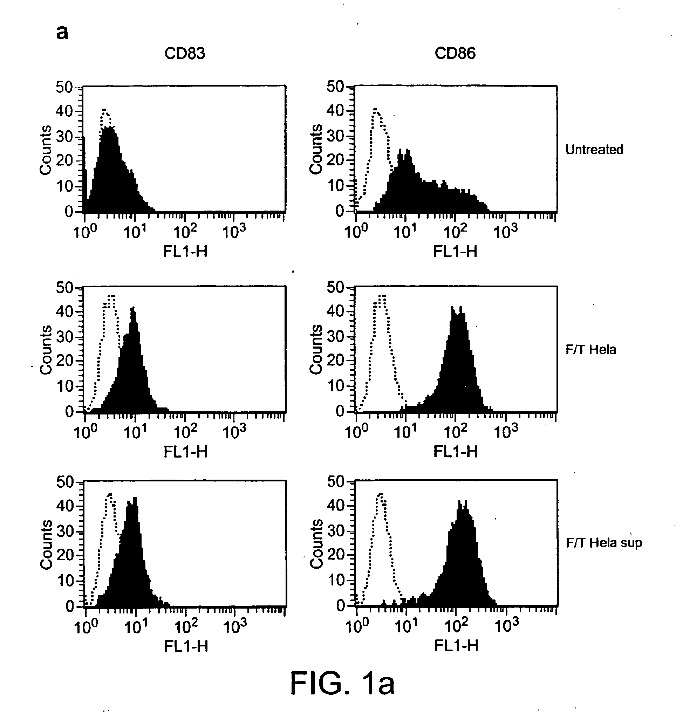

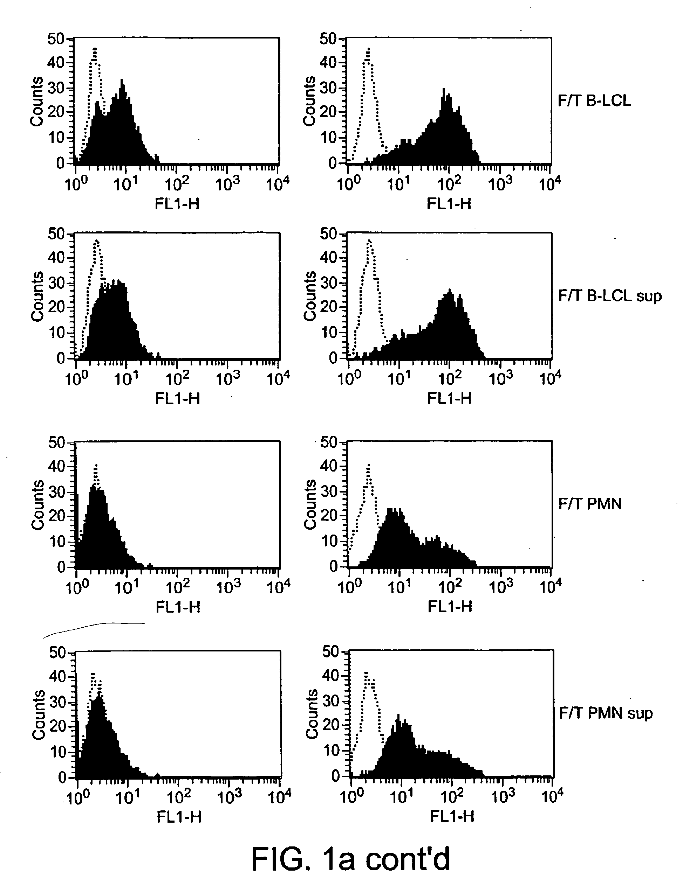

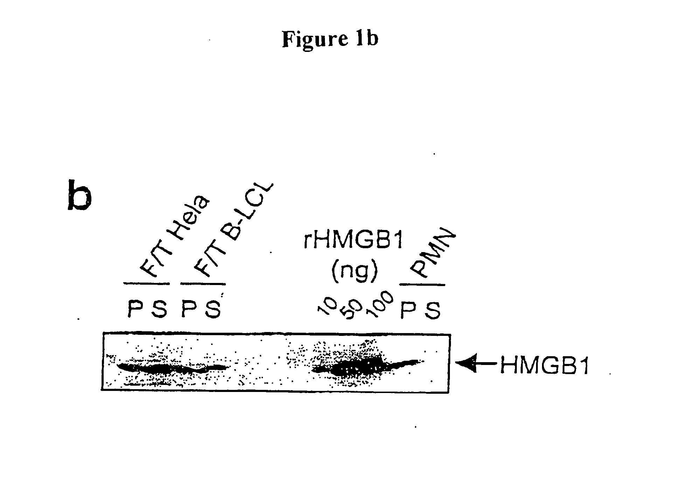

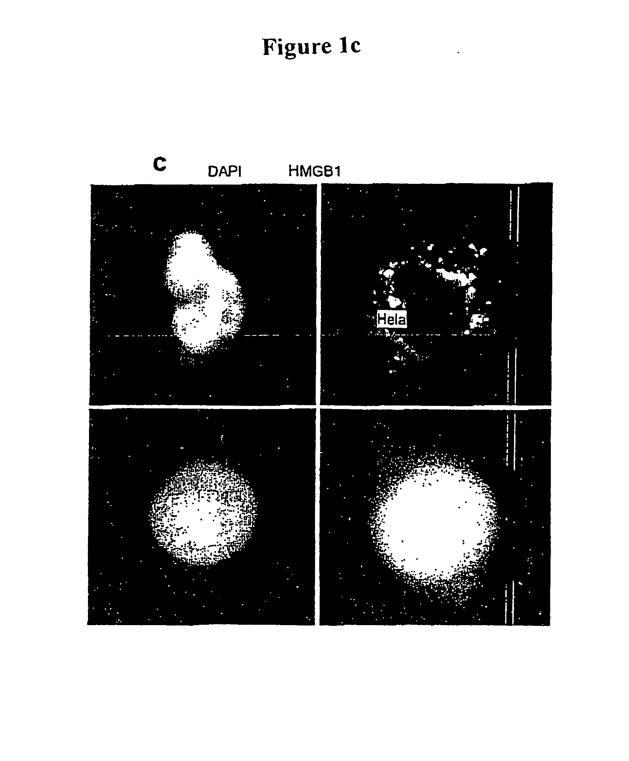

**Fig.1**: The supernatants of necrotic cells that cause maturation of DCs contain HMGB1.
   a. Expression of the CD83 and CD86 surface molecules was evaluated by flow cytometry on untreated immature DCs or on DCs stimulated with Hela, B-LCL and frozen/thawed (F/T) PMNs, or with their supernatants (sup).
   b. HMGB1 was evaluated by western blotting in the pellet (P) or in the supernatant (S) of HeLa, B-LCL or necrotic F/T PMNs. Purified recombinant HMGB1 was used as control (rHMGB1; 10, 50 or 100 ng/lane).
   c. The intracellular distribution of HMGB1 was evaluated by immunohistochemistry in PMN and HeLa cells (panels on right). The nuclei were revealed by DAPI staining (panels on left).

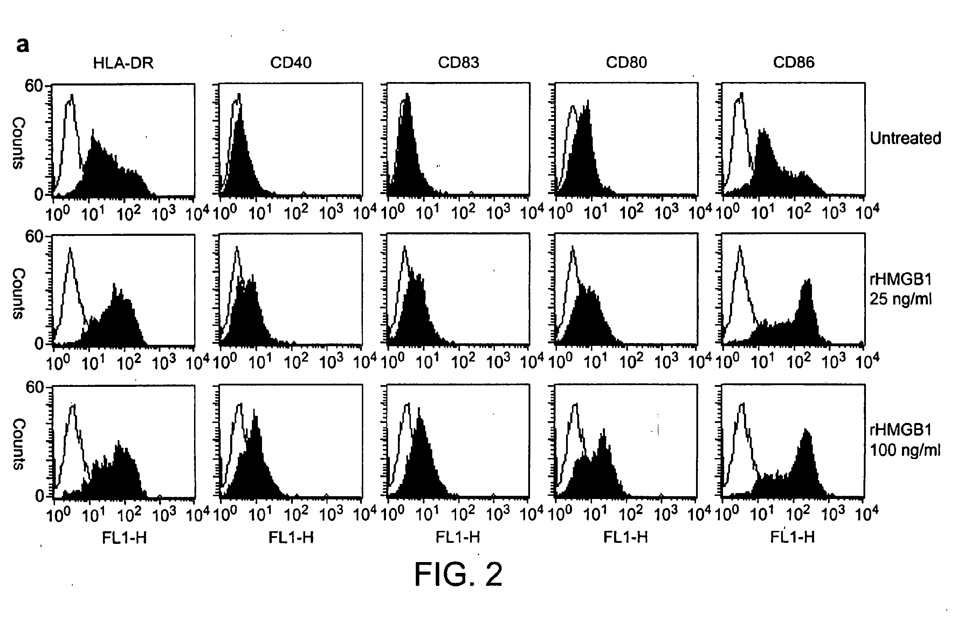

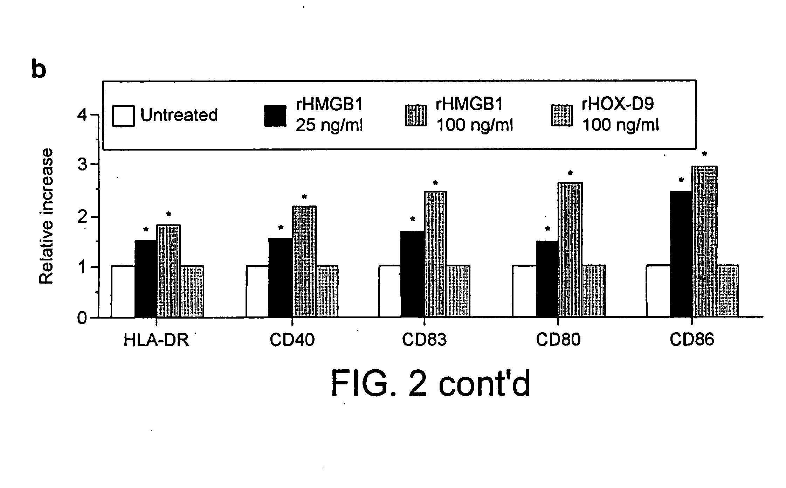

**Fig. 2****:** HMGB1 is sufficient to induce the maturation of DCs.
   a. Expression of the surface molecules HLA-DR, CD40, CD83, CD80 and CD86 was evaluated by flow cytometry on untreated immature DCs or on DCs treated with rHMGB1 (25 or 100 ng/ml).
   b. The relative increase in surface expression is expressed as the mean fluorescence intensity (MFI) of cells treated with rHMGB1 relative to the MFI of untreated immature DC cells.
      The experiments were repeated at least three times with DCs from different donors. The differences were statistically significant, on the basis of the Kolmogorov-Smirnov algorithms (*=D/s(n) values > 25).

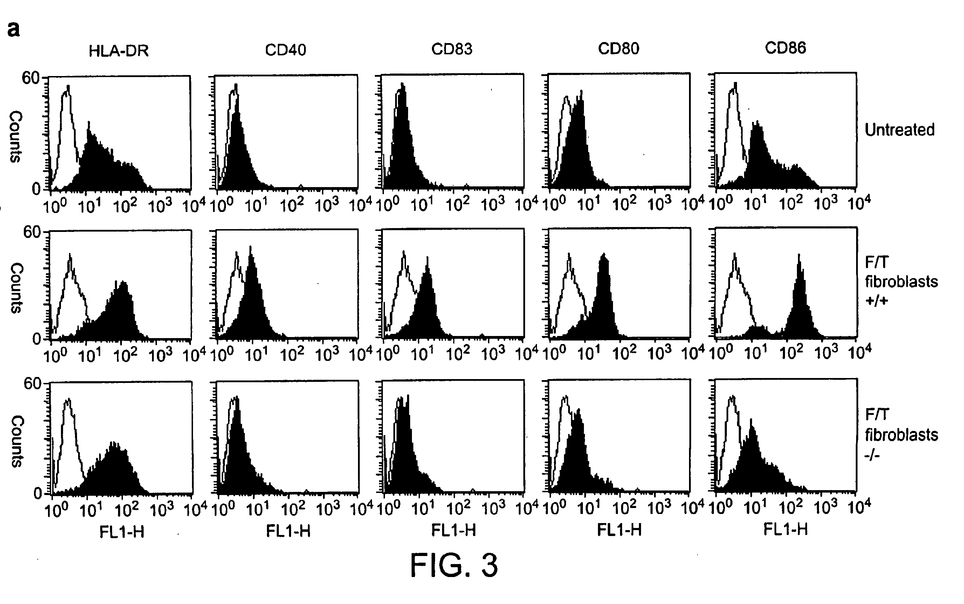

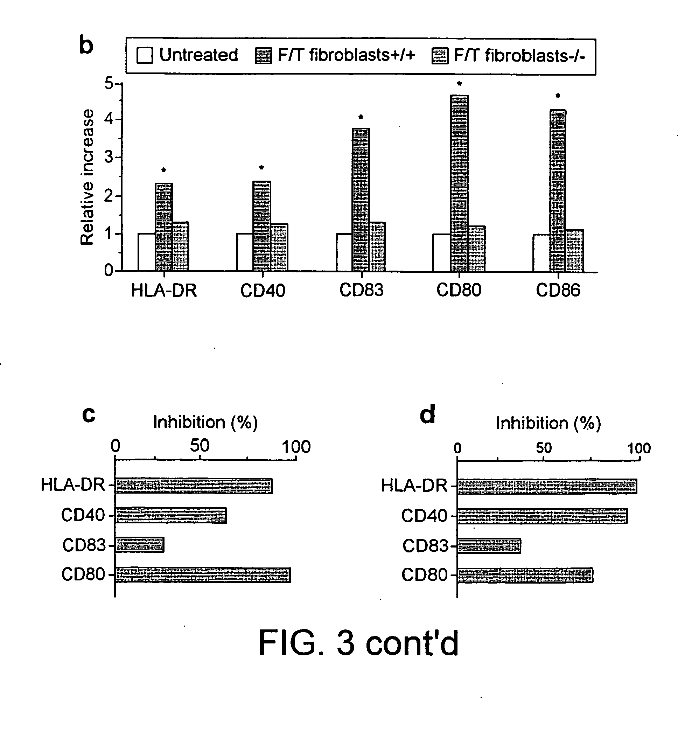

**Fig. 3****:** HMGB1 is necessary for the maturation of DCs induced by necrotic cells.
   a. Expression of the surface molecules HLA-DR, CD40, CD83, CD80 and CD86 was evaluated by flow cytometry on untreated immature DCs or on DCs treated with the supernatants of necrotic wild-type fibroblasts (F/T +/+) or of the corresponding *Hmgb1-l-* fibroblasts.
   b. The relative increase in surface expression is expressed as the mean fluorescence intensity (MFI) of DCs treated with the supernatants of *Hmgb1* +/+ necrotic fibroblasts or of DCs treated with the supernatants of *Hmgb1 -*/*-* necrotic fibroblasts relative to the MFI of untreated immature DCs. The experiments were repeated at least three times with DCs from various donors. Only the DCs treated with supernatants of *Hmgb1* +/+ necrotic fibroblasts were significantly higher, on the basis of the Kolmogorov/Smimov algorithm (*=D/s (n) values > 25).
   c. Antibodies specific for HMGB1 inhibit the maturation of DCs induced by rHMGB1 (25 ng/ml). The results are expressed as percentage inhibition, calculated as follows: inhibition % = 100 x (1 - MFI of DCs treated with rHMGB1 in the presence of anti-HMGB1 antibodies / MFI of DCs treated with rHMGB1 without anti-HMGB1 antibodies).
   d. Antibodies specific for HMGB1 inhibit the maturation of DCs induced by the supernatants of necrotic F/T B-LCL. The results are expressed as percentage inhibition, calculated as follows: inhibition % = 100 x (1 - MFI of DCs treated with supernatants of necrotic F/T B-LCL in the presence of anti-HMGB1 antibodies / MFI of DCs treated with supernatants of necrotic F/T B-LCL without anti-HMGB1 antibodies).

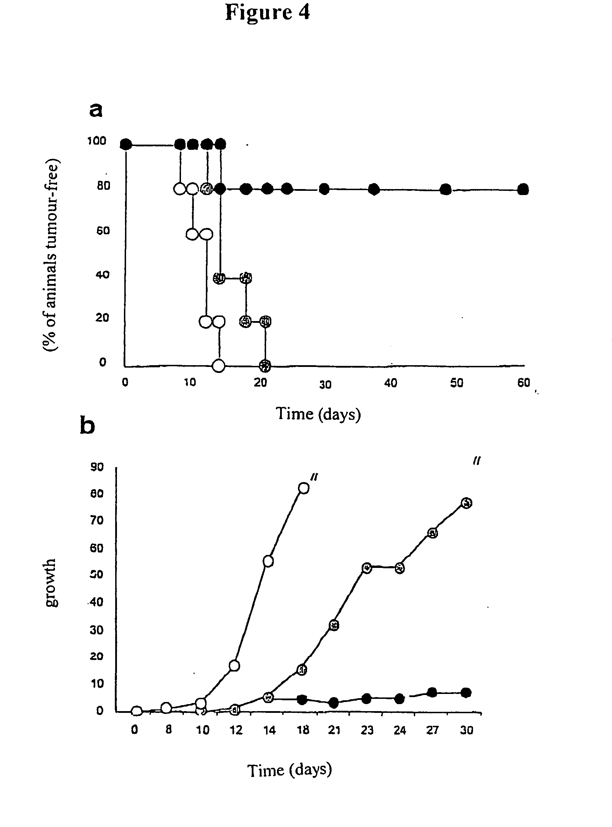

**Fig. 4****:** HMGB1 increases the immunogenicity of apoptotic lymphoma cells.
   The subcutaneous growth of RMA lymphoma cells was evaluated in groups of five C57BL/6 mice vaccinated with PBS (empty circles), 1x10⁶ apoptotic RMA cells (light filled circles) or 1x10⁶ apoptotic RMA cells in the presence of rHMGB1 (0.5 µg/mouse - dark filled circles).
   a. The results shown (y axis) indicate the fraction of mice without tumours at different times after administration of live lymphoma cells (x axis).
   b. The results shown (y axis) indicate the average diameter of the tumour at different times after administration of live lymphoma cells (x axis).

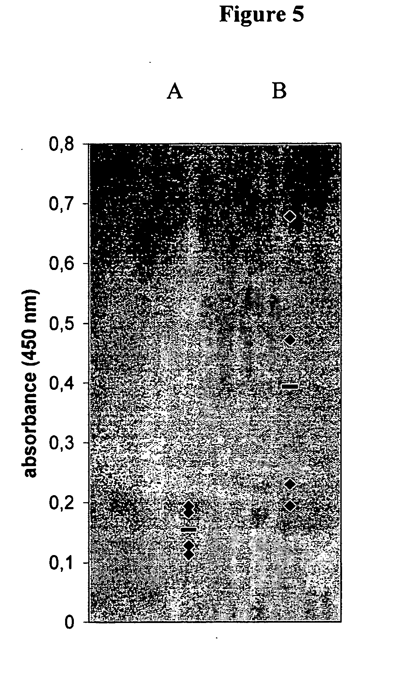

**Fig.5****:** Ovalbumin specific IgM antibodies detected by ELISA 10 days after immunisation with the soluble antigen in the abosence (A) or in the presence (B) of recombinant purified HMGB 1.

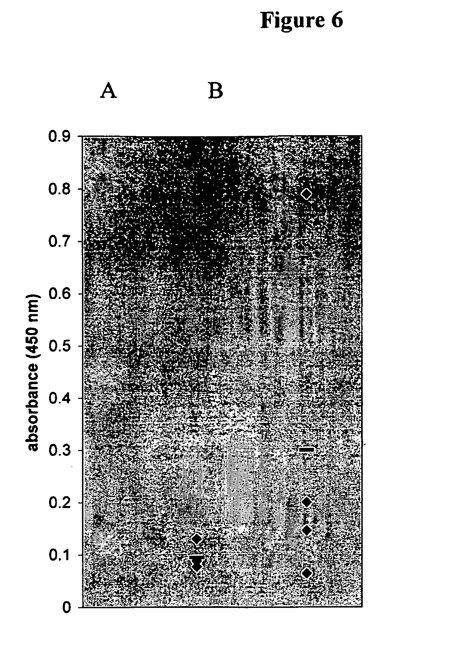

**Fig.6****:** Ovalbumin specific IgG1 antibodies detected by ELISA 10 days after immunisation with the soluble antigen in the abosence (A) or in the presence (B) of recombinant purified HMGB 1.

### Examples

It has now been found, surprisingly, that HMGB1 possesses a pronounced effect of induction of the maturation of dendritic cells.

In confirmation of this, it has been seen that HMGB1 accumulates in the culture medium of necrotic cells that induce maturation of DCs, such as B-LCL or HeLa. In contrast, the culture medium of necrotic polymorphonuclear cells, not containing HMGB1, does not induce activation of the said dendritic cells.

Firstly we evaluated whether HMGB1 released from necrotic cells is necessary for the maturation of DCs. For this purpose, dendritic cells were stimulated with the culture medium of mouse embryo fibroblasts and with corresponding media of *Hmgb1* -/- cells. Only the media of necrotic fibroblasts containing HMGB1 induced the maturation of DCs. The specific effect of HMGB1 was demonstrated using recombinant HMGB1, which was able to induce maturation to the same extent as the necrotic cells. It was found, however, that neither the recombinant protein, nor the culture media of necrotic cells, induced activation of DCs in the presence of HMGB1 neutralizing antibodies.

Then the capacity of HMGB1 to induce maturation of antigen-presenting DCs *in vivo* was evaluated. As expected, since the apoptotic lymphoma cells are in themselves barely immunogenic (because they are recognized as "self" by mice that are syngeneic with them), all the mice inoculated with apoptotic RMA cells developed the tumour. In contrast, 80% of the animals immunized with lymphoma cells in the presence of HMGB1 rejected the tumour, whereas in the other 20% neoplastic growth was greatly inhibited.

Taken together, these results show that HMGB1 activates the antigen-presenting cells *in vitro* and *in vivo.* It is believed that HMGB1, once released by the necrotic cells, supplies the DCs with the signal necessary for maturation, and for subsequent migration to the lymph nodes and initiation of the immune response.

### Example 1 - Cells

DC cells and neutrophils were obtained from the blood of healthy donors, as described (26). Embryonic fibroblasts were obtained from wild-type and *Hmgb1*-/- animals. All the lines were tested for contamination by mycoplasma using PCR.

### Example 2 - Apoptosis and necrosis

The cells were killed by necrosis as a result of three cycles of freezing/thawing, as described (4). Actual death was confirmed by FACS analysis after staining with FITC-annexin V and propidium iodide (9, 26). Apoptosis was induced by UV radiation (9).

### Example 3 - Maturation of DCs

Immature DC cells were stimulated with apoptotic or necrotic cells (ratio DCs:dead cells = 2:1) or with their culture medium. Where indicated, the experiments were conducted in the presence of or in the absence of anti-HMGB1 polyclonal antibodies (Pharmingen). In parallel experiments, the DCs were incubated with purified HMGB1 (0.1-100 ng/ml) or with the protein HOXD9. Maturation was evaluated after 48 hours on the basis of morphological findings and flow cytometry (9). Cellular vitality was evaluated at various treatment times. To exclude possible contamination by endotoxin, experiments were carried out in a medium containing polymyxin (Sigma; 70 u/ml), as described (12). In these conditions, addition of purified LPS (E. coli 026:B6 from Sigma, 100 ng/ml) did not lead to significant maturation, whereas recombinant TNFα caused efficient maturation of the DCs.

### Example 4 - Production and detection of HMGB1

The pT7-7-rHMGB1cm plasmid was used for expression of HMGB1 of full length in *E. coli* strain BL21(-). Then the protein was purified (10). Extracts of dying cells obtained with detergent, or the supernatants, transferred onto nitrocellulose membranes, were probed with specific anti-HMGB1 polyclonal antibodies and anti-rabbit antibodies conjugated with FITC (Boehringer) (10). The nuclei were counter-stained with DAPI (10).

### Example 5 - Immunisation

C57BL/6 mice were injected subcutaneously twice a week with PBS or 1x10⁶ apoptotic RMA cells, in the presence of or in the absence of recombinant HMGB1 (0.5 µg/mouse). Contamination with endotoxin, assessed with the kinetic test "QLC Limulus amebocyte cell lysate" (BioWhittaker, Walkersville, MD), was less than 0.03 U/animal. After 14 days the mice were stimulated subcutaneously in the opposite flank with 50x10³ live lymphoma RMA cells. The appearance and size of the tumour were evaluated as described (18).

### Example 6 - Evaluation of the adjuvant effect of HMGB1 on the production of antibodies directed against soluble antigens.

Vaccination procedures require the availability of adjuvant signals to elicit protective immune responses. Data suggest that the nuclear constituent HMGB1 delivers an adjuvant signal when administered in association with particulate antigens (i.e. dying tumor cells). Here we analyze whether HMGB1 may indeed favor the production of antibodies against soluble antigens. To this aim we vaccinated experimental animals with a nominal antigen (chicken egg albumin, OVA) and after ten days, evaluated the concentration of OVA-specific immunoglobulins.

C57B1/6 (B6) mice were purchased and maintained and bred in the SPF unit of our Institution. 5 weeks old animal were immunized, and re-boosted 20 days after. All immunizations were done by sc injection in the foot-pad of 20 µg of ovalbumin/mouse (Sigma), administrated in soluble form (in PBS) either when given alone or when mixed with 1 µg/mouse of recombinant purified HMGB 1.

For serum analysis by ELISA of IgM and IgG1, wells were coated with 2 mg/ml OVA and exposed to serial dilutions of serum samples from each single immunized animal. Sera used were obtained 10 days after immunisation. OVA specific antibodies were revealed using isotype-specific polyclonal goat anti-mouse antibodies conjugated to alkaline phosphatase (AP) (all from Southern Biotechnology Associate Inc., Birmingham, AL). Plates were washed before addition of a 3,3,5,5-tetramethylbenzidine substrate solution. The reaction was stopped with 0.5 M H2SO4 and the OD read at 450 nm.

Mice immunized with OVA in the presence of HMGB1 developed higher titres of OVA-specific immunoglobulins of the IgM and IgG1 isotype.

The present invention relates to the ability of HMGB1 to behave as an adjuvant for pathogens, including tumors, bacteria and viruses. The ability to increase the humoral antibody response is critical for most vaccination procedures against infectious agents. These vaccinations rely on the injection of antigens in a recipient, to elicit antibodies able i) to directly bind to the pathogens and inactivate it or ii) to block the pathogen ability to invade cells of the host. The latter step is for example crucial for the development of "preventive" vaccines against difficult pathogens, like HIV or HCV.

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in molecular biology or related fields are intended to be within the scope of the following claims.

### References

1. Janeway, C.A. The immune system evolved to discriminate infectious nonself from noninfectious nonself. Immunol Today. 13, 11-16 (1992).
2. Banchereau, J. & Steinman, R.M. Dendritic cells and the control of immunity. Nature 392, 245-252 (1998).
3. Gallucci, S., Lolkema, M. & Matzinger, P. Natural adjuvants: endogenous activators of dendritic cells. Nat Med. 5, 1249-1255 (1999).
4. Sauter, B., Albert, M.L., Francisco, L., Larsson, M., Somersan, S. & Bhardwaj, N. Consequences of cell death: exposure to necrotic tumor cells, but not primary tissue cells or apoptotic cells, induces the maturation of immunostimulatory dendritic cells. J. Exp. Med. 191, 423-434 (2000).
5. Ignatius, R., Marovich, M., Mehlhop, E., Villamide, L., Mahnke, K., Cox, W.I., Isdell, F., Frankel, S.S., Mascola, J.R., Steinman, R.M. & Pope, M. Canarypox virus-induced maturation of dendritic cells is mediated by apoptotic cell death and tumor necrosis factor alpha secretion. J. Virol. 74, 11329-11338 (2000).
6. Shi, Y., Zheng, W. & Rock, K.L. Cell injury releases endogenous adjuvants that stimulate cytotoxic T cell responses. Proc. Natl. Acad. Sci. U.S.A. 97, 14590-14595. (2000).
7. Basu, S., Binder, R.J., Suto, R., Anderson, K.M. & Srivastava, P.K. Necrotic but not apoptotic cell death releases heat shock proteins, which deliver a partial maturation signal to dendritic cells and activate the NF-kappa B pathway. Int. Immunol. 12, 1539-1546 (2000).
8. Larsson, M., Fonteneau, J.F. & Bhardwaj, N. Dendritic cells resurrect antigens from dead cells. Trends Immunol. 3, 141-148 (2001).
9. Rovere, P., Vallinoto, C., Bondanza, A., Crosti, M.C., Rescigno, M., Ricciardi-Castagnoli, P., Rugarli, C. & Manfredi, A.A. Bystander apoptosis triggers dendritic cell maturation and antigen-presenting function. J. Immunol. 161, 4467-4471 (1998).
10. Scaffidi, P., Misteli, T. & Bianchi, M.E. Retention of HMGB1, a nuclear protein that mediates inflammation, distinguishes apoptosis from necrosis. Submitted to Nature.
11. Yang, H., Wang. H. & Tracey K.J. HMG-1 rediscovered as a cytokine. Shock 15, 247-253 (2001).
12. Andersson, U., Wang, H., Palmblad, K., Aveberger, A.C., Bloom, O., Erlandsson-Harris, H., Janson, A., Kokkola, R., Zhang, M., Yang, H. & Tracey, K.J. High Mobility Group 1 Protein (HMG-1) stimulates proinflammatory cytokine synthesis in human minocytes. J. Exp. Med. 192, 565-570 (2000).
13. Wang, H., Bloom, O., Zhang, M., Vishnubhakat, J.M., Ombrellino, M., Che, J., Frazier, A., Yang, H., Ivanova, S., Borovikova, L., Manogue, K.R., Faist, E., Abraham, E., Andersson, J., Andersson, U., Molina, P.E., Abumrad, N.N., Sama, A. & Tracey, K.J. HMG-1 as a late mediator of endotoxin lethality in mice. Science 285, 248-251 (1999).
14. Ombrellino, M., Wang, H., Ajemian, M.S., Talhouk, A., Scher, L.A., Friedman, S.G., Tracey, K.J. Increased serum concentrations of high-mobility-group protein 1 in haemorrhagic shock. Lancet 354, 1446-1447 (1999).
15. Austyn, J.M. Death, destruction, danger and dendritic cells. Nat Med. 5, 1232-1233 (1999).
16. Gallucci, S. & Matzinger, P. Danger signals: SOS to the immune system. Current Opin. Immunol. 13, 114-119 (2001).
17. Calogero, S., Grassi, F., Aguzzi, A., Voigtlander, T., Ferrier, P., Ferrari, S., Bianchi, M.E. The lack of chromosomal protein Hmg1 does not disrupt cell growth but causes lethal hypoglycaemia in newborn mice. Nat Genet. 22, 276-280 (1999).
18. Ronchetti, A., Rovere, P., Iezzi, G., Galati, G., Heltai, S., Protti, M.P., Garancini, M.P., Manfredi, A.A., Rugarli, C., Bellone M. Immunogenicity of apoptotic cells in vivo: role of antigen load, antigen-presenting cells, and cytokines. J Immunol. 163, 130-136 (1999).
19. Albert ML, Sauter B, Bhardwaj N. Dendritic cells acquire antigen from apoptotic cells and induce class I-restricted CTLs. Nature 392, 86-89 (1998).
20. Inaba. K., Turley, S., Yamaide, F., Iyoda, T., Mahnke, K., Inaba, M., Pack, M., Subklewe, M., Sauter, B., Sheff, D., Albert, M., Bhardwaj, N., Mellman, I. & Steinman, R.M. Efficient presentation of phagocytosed cellular fragments on the major histocompatibility complex class II products of dendritic cells. J. Exp. Med. 188, 2163-2173 (1998).
21. Kurts, C., Miller, J.F.A.P., Subramaniam, R.M., Carbone, F.R. & Heath, W.R. MIIC class I restricted cross presentation is biased towards high dose antigens and those released during cellular destruction. J. Exp. Med. 188, 409-414 (1998).
22. Steinman, R.M., Turley, S., Mellman, I., Inaba, K. The induction of tolerance by dendritic cells that have captured apoptotic cells. J. Exp. Med. 191, 411-416 (2000).
23. Savill, J. & Fadok, V. Corpse clearance defines the meaning of cell death. Nature 407, 784-788 (2000).
24. Platt, N., da Silva, R.P & Gordon, S. Recognising death: the phagocytosis of apoptotic cells. Trends Cell Biol. 8, 365-372 (1998).
25. Gregory, C.D. CD14-dependent clearance of apoptotic cells: relevance to the immune system. Current Opin. Immunol. 12,27-34 (2000).
26. Rovere, P., Peri, G., Fazzini, F., Bottazzi, B., Doni, A., Bondanza, A., Zimmermann, V.S., Garlanda, C., Fascio, U., Sabbadini, M.G., Rugarli, C., Mantovani, A. & Manfredi, A.A. The long pentraxin PTX3 binds to apoptotic cells and regulates their clearance by antigen presenting dendritic cells. Blood 96, 4300-4306 (2000).
27. Aderem A, Ulevitch RJ., Toll-like receptors in the induction of the innate immune response. Nature 17; 406(6797): 782-7 (Aug 2000).
28. Ogata H, Su I, Miyake K, Nagai Y, Akashi S, Mecklenbrauker I, Rajewsky K, Kimoto M, Tarakhovsky A. The toll-like receptor protein RP105 regulates lipopolysaccharide signalling in B cells. J Exp Med. 2000 Jul 3; 192(1): 23-9.

## Claims

1. Use of the protein HMGB1 or a biologically active fragment thereof, or a polynucleotide encoding therefor, for the preparation of a medicament for inducing the activation of an antigen presenting cell (APC), and wherein the medicament is for stimulating an immune response.

2. Use according to claim 1 where the said APC is a dendritic cell.

3. Use according to claim 1 or 2 wherein the activation is carried out *in vitro.*

4. Use according to claim 1 wherein the said medicament is in the form of a vaccine.

5. Use according to claim 4 wherein the vaccine is for use in relation to a tumor or bacterial or viral infection.

6. Use according to any one of claims 1 to 5 wherein the said medicament is in the form of an adjuvant.

7. Use according to any preceding claim wherein the medicament further comprises an antigen.

8. Use according to any preceding claim wherein the antigen is a tumor, bacterial or viral antigen.

9. Use of an antibody or antisense sequence of the protein HMGB1 or a biologically active fragment thereof, or a polynucleotide encoding therefor, for the preparation of a medicament for reducing activation of an APC.

10. Use according to claim 9 wherein said reducing activation is carried out *in vitro.*

11. Use according to claim 9 wherein said reducing activation downregulates an antigen specific immune response.

12. Use according to claim 9 wherein said reducing activation is for the treatment of an inflammatory or autoimmune disease, allergy or transplant rejection.

13. Use according to claim 11 or 12 wherein the medicament is in the form of a vaccine.

14. An *in vitro* method for producing an activated APC comprising exposing the APC to the protein HMGB1 or a biologically active fragment thereof, or a polynucleotide encoding therefor.

15. A method according to claim 14 wherein the APC is also exposed to an antigen.

16. A method according to claim 14 or 15 wherein the APC and/or antigen are also exposed to a T cell.

17. A method according to claim 15 or 16 wherein the antigen is a tumor, bacterial or viral antigen.

18. An *in vitro* method of reducing or preventing activation of an APC comprising exposing the APC to an antibody or antisense sequence of the protein HMGB1 or a biologically active fragment therefor, or a polynucleotide encoding therefor.

19. A method according to claim 18 wherein the APC is also exposed to an antigen.

20. A method according to claims 18 or 19 wherein the APC and/or antigen are also exposed to a T cell.

21. A method according to claim 19 or 20 wherein the antigen is an allergen or is associated with an inflammatory condition, autoimmune disease or transplant rejection.

22. A method according to any one of claims 18 to 21 wherein the APC is transfected with a vector for the expression of an antigen or of an MHC molecule.

23. A method according to any one of claims 14 to 22 wherein the APC is a dendritic cell.

24. A method according to any one of claims 14 to 23 wherein the HMGB1 is in the form of a vaccine.

25. A pharmaceutical composition comprises the protein HMGB1 or a biologically active fragment thereof or a polynucleotide encoding therefor and an antigen.

26. A pharmaceutical composition according to claim 25, in the form of a vaccine.

27. A pharmaceutical composition according to claim 25 or 26 further comprising an APC.

## Patentansprüche

1. Verwendung des Proteins HMGB1 oder eines biologisch aktiven Fragments davon oder eines dafür kodierenden Polynukleotids für die Herstellung eines Medikaments zur Induktion der Aktivierung einer Antigen-präsentierenden Zelle (APC), wobei das Medikament der Stimulierung einer Immunantwort dient.

2. Verwendung gemäß Anspruch 1, wobei die APC eine dendritische Zelle ist.

3. Verwendung gemäß Anspruch 1 oder 2, wobei die Aktivierung *in vitro* ausgeführt wird.

4. Verwendung gemäß Anspruch 1, wobei das Medikament in Form eines Impfstoffs vorliegt.

5. Verwendung gemäß Anspruch 4, wobei der Impfstoff zur Verwendung in Bezug auf einen Tumor oder eine bakterielle oder virale Infektion dient.

6. Verwendung gemäß einem der Ansprüche 1 bis 5, wobei das Medikament in der Form eines Zusatzstoffes vorliegt.

7. Verwendung gemäß einem der vorangehenden Ansprüche, wobei das Medikament weiter ein Antigen umfasst.

8. Verwendung gemäß einem der vorangehenden Ansprüche, wobei das Antigen ein Tumor-, bakterielles oder virales Antigen ist.

9. Verwendung eines Antikörpers oder einer Antisense-Sequenz des Proteins HMGB1 oder eines biologisch aktiven Fragments davon oder eines dafür kodierenden Polynukleotids für die Herstellung eines Medikaments zur Reduzierung der Aktivierung einer APC.

10. Verwendung gemäß Anspruch 9, wobei die Reduzierung der Aktivierung *in vitro* ausgeführt wird.

11. Verwendung gemäß Anspruch 9, wobei die Reduzierung der Aktivierung eine Antigen-spezifische Immunantwort abschwächt.

12. Verwendung gemäß Anspruch 9, wobei die Reduzierung der Aktivierung der Behandlung einer inflammatorischen oder Autoimmun-Erkrankung, einer Allergie oder Transplantatabstoßung dient.

13. Verwendung gemäß Anspruch 11 oder 12, wobei das Medikament in der Form eines Impfstoffs vorliegt.

14. *In vitro*-Verfahren zur Herstellung einer aktivierten APC, bei dem man die APC dem Protein HMGB1 oder einem biologisch aktiven Fragment davon oder einem dafür kodierenden Polynukleotid aussetzt.

15. Verfahren gemäß Anspruch 14, wobei die APC auch einem Antigen ausgesetzt wird.

16. Verfahren gemäß Anspruch 14 oder 15, wobei die APC und/oder ein Antigen auch einer T-Zelle ausgesetzt werden.

17. Verfahren gemäß Anspruch 15 oder 16, wobei das Antigen ein Tumor-, bakterielles oder virales Antigen ist.

18. *In vitro*-Verfahren zur Reduzierung oder zum Verhindern der Aktivierung einer APC, bei dem man die APC einem Antikörper oder einer Antisense-Sequenz des Proteins HMGB1 oder einem biologisch aktiven Fragment davon oder einem dafür kodierenden Polynukleotid aussetzt.

19. Verfahren gemäß Anspruch 18, wobei die APC auch einem Antigen ausgesetzt wird.

20. Verfahren gemäß Anspruch 18 oder 19, wobei die APC und/oder ein Antigen auch einer T-Zelle ausgesetzt werden.

21. Verfahren gemäß Anspruch 19 oder 20, wobei das Antigen ein Allergen ist oder mit einem inflammatorischen Zustand, Autoimmun-Erkrankung oder Transplantatabstoßung assoziiert wird.

22. Verfahren gemäß einem der Ansprüche 18 bis 21, wobei die APC mit einem Vektor für die Expression eines Antigens oder eines MHC-Moleküls transfiziert wird.

23. Verfahren gemäß einem der Ansprüche 14 bis 22, wobei die APC eine dendritische Zelle ist.

24. Verfahren gemäß einem der Ansprüche 14 bis 23, wobei das HMGB1 in der Form eines Impfstoffs vorliegt.

25. Pharmazeutische Zusammensetzung, welche das Protein HMGB1 oder ein biologisch aktives Fragment davon oder ein dafür kodierendes Polynukleotid und ein Antigen umfasst.

26. Pharmazeutische Zusammensetzung gemäß Anspruch 25 in der Form eines Impfstoffs.

27. Pharmazeutische Zusammensetzung gemäß Anspruch 25 oder 26, die weiter eine APC umfasst.

## Revendications

1. Utilisation de la protéine HMGB1 ou d'un fragment biologiquement actif de celle-ci, ou d'un polynucléotide codant pour celle-ci, pour la préparation d'un médicament induisant l'activation d'une cellule présentatrice de l'antigène (CPA), ce médicament ayant pour but de stimuler une réponse immunitaire.

2. Utilisation, conformément à la revendication 1, selon laquelle ladite CPA est une cellule dendritique.

3. Utilisation, conformément aux revendications 1 ou 2, selon laquelle l'activation est réalisée *in vitro.*

4. Utilisation, conformément à la revendication 1, selon laquelle ledit médicament est sous la forme d'un vaccin.

5. Utilisation, conformément à la revendication 4, selon laquelle le vaccin est destiné à être utilisé en relation avec une tumeur ou une infection bactérienne ou virale.

6. Utilisation, conformément aux revendications 1 à 5, où ledit médicament est sous la forme d'un adjuvant.

7. Utilisation, conformément à toute revendication précédente, selon laquelle le médicament comprend en outre un antigène.

8. Utilisation conformément à toute revendication précédente selon laquelle l'antigène est une tumeur, un antigène bactérien ou viral.

9. Utilisation d'un anticorps ou d'une séquence antisens de la protéine HMGB1 ou d'un fragment biologiquement actif de celle-ci, ou d'un polynucléotide codant pour celle-ci, pour la préparation d'un médicament réduisant l'activation d'une CPA.

10. Utilisation, conformément à la revendication 9, selon laquelle ladite activation réduite est réalisée *in vitro*.

11. Utilisation, conformément à la revendication 9, selon laquelle ladite activation réduite induit la diminution d'une réponse immunitaire spécifique de l'antigène.

12. Utilisation, conformément à la revendication 9, selon laquelle ladite activation réduite est destinée au traitement d'une maladie auto-immune ou inflammatoire, d'allergie ou de rejet de greffe.

13. Utilisation, conformément aux revendications 11 et 12, selon laquelle ledit médicament est sous forme d'un vaccin.

14. Une méthode *in vitro* afin de produire une CPA activée comprenant en l'exposition d'une CPA à la protéine HMGB1 ou à un fragment biologiquement actif de celle-ci ou à un polynucléotide codant pour celle-ci.

15. Une méthode, conformément à la revendication 14, selon laquelle la CPA est également exposée à un antigène.

16. Une méthode, conformément aux revendications 14 et 15, selon laquelle la CPA et/ou l'antigène sont également exposés à une cellule T.

17. Une méthode, conformément aux revendications 15 et 16, selon laquelle l'antigène est une tumeur, un antigène bactérien ou viral.

18. Une méthode *in vitro* afin de réduire ou de prévenir l'activation d'une CPA comprenant en l'exposition d'une CPA à un antigène ou à une séquence antisens de la protéine HMGB1 ou à un fragment biologiquement actif de celle-ci ou à un polynucléotide codant pour celle-ci.

19. Une méthode, conformément à la revendication 18, selon laquelle la CPA est également exposée à un antigène.

20. Une méthode, conformément aux revendications 18 et 19, selon laquelle la CPA et/ou un antigène sont également exposés à une cellule T.

21. Une méthode, conformément aux revendications 19 et 20, selon laquelle l'antigène est un allergène ou est associé à une condition inflammatoire, à une maladie auto-immune ou à un rejet de greffe.

22. Une méthode, conformément aux revendications 18 à 21, selon laquelle la CPA est transfectée avec un vecteur afin d'inuire l'expression d'un antigène ou d'une molécule du CMH.

23. Une méthode, conformément aux revendications 14 à 22, selon laquelle la CPA est une cellule dendritique.

24. Une méthode, conformément aux revendications 14 à 23, selon laquelle la protéine HMGB1 est sous forme d'un vaccin.

25. Une composition pharmaceutique comprenant la protéine HMGB1 ou un fragment biologiquement actif de celle-ci ou un polynucléotide codant pour celle-ci, et un antigène.

26. Une composition pharmaceutique, conformément à la revendication 25, sous la forme d'un vaccin.

27. Une composition pharmaceutique, conformément aux revendications 25 ou 26, comprenant en plus une CPA.
